# FASCICULE DE BREVET EUROPEEN

(11) **EP 0 971 687 B1**
(45) Date de publication et mention de la délivrance du brevet: **07.04.2004**
(21) Numéro de dépôt: 98943988.0
(22) Date de dépôt: 15.09.1998
(51) Int. Cl.: A61K 7/48

(54) **SOLUTIONS AQUEUSES DE DERIVES D'ACIDE SALICYLIQUE**
WÄSSRIGE LÖSUNGEN VON SALICYLSAURE DERIVATEN
AQUEOUS SOLUTIONS OF SALICYLIC ACID DERIVATIVES

(30) Priorité: 16.09.1997 US 931309
(43) Date de publication de la demande: 19.01.2000
(73) Titulaire: L'OREAL, 75008 Paris (FR)
(72) Inventeur: PINZON, Carlos, Hackensack, NJ 07601 (US)
(74) Mandataire: Rasson, Catherine
(86) Numéro de dépôt international: PCT/FR1998/001969
(87) Numéro de publication internationale: WO 1999/013857

(56) Documents cités:
- EP-A- 0 615 748
- EP-A- 0 738 510
- EP-A- 0 765 658
- WO-A-95/03032
- WO-A-95/03779
- GB-A- 2 283 421
- US-A- 5 382 432

## Description

L'invention porte sur les solutions aqueuses de dérivés d'acide salicylique, utiles en tant que compositions cosmétiques et/ou dermatologiques. Ces solutions aqueuses de dérivés d'acide salicylique sont, de préférence, transparentes, non troubles et stables à long terme.

L'utilisation de dérivés d'acide salicylique en tant qu'agents kératolytiques pour le traitement de l'acné et en tant qu'agents anti-vieillissement est connue. Les documents FR 2 581 542 et EP 378 936 décrivent ces dérivés. Cependant, les dérivés de l'acide salicylique sont, en général, présents sous forme cristalline et ils ne sont pas suffisamment solubles dans l'eau ou les huiles qui sont utilisées traditionnellement dans le champ cosmétique et dermatologique Par conséquent, les dérivés d'acide salicylique ont tendance à demeurer cristallins dans différentes compositions, ce qui réduit considérablement la biodisponibilité du composé. En outre, la présence de dérivés cristallins d'acide salicylique offre des compositions qui ne sont pas stables en raison, notamment, de la sédimentation, et qui sont de texture et d'aspect désagréables du point de vue du consommateur.

Par conséquent, un objet de la présente invention consiste à offrir des compositions aqueuses stables de dérivés d'acide salicylique, utiles en tant que compositions cosmétiques et/ou dermatologiques.

Un autre objet de la présente invention consiste à offrir des solutions aqueuses stables de dérivés d'acide salicylique.

Un autre objet de la présente invention consiste à offrir des compositions cosmétiques et/ou dermatologiques qui contiennent des dérivés d'acide salicylique, formulées de manière à être acceptables pour le consommateur et être, de préférence, stables et transparentes.

Un autre objet de la présente invention consiste à offrir une méthode de traitement ou de prévention des signes du vieillissement (rides, apparence de lignes fines, pigmentation, etc.) (ci-après les "caractéristiques cutanées") par application sur la caractéristique cutanée et, en option, sur la peau avoisinante, des solutions, compositions, mélanges et formulations aqueux de dérivés d'acide salicylique faisant l'objet de l'invention.

Un autre objet encore de la présente invention consiste en l'utilisation des compositions précitées pour préparer une composition dermatologique destinée à traiter et/ou à prévenir les verrues et/ou l'acné.

A ces fins et autres fins sont fournies des solutions aqueuses de dérivés d'acide salicylique comprenant, parmi d'autres composants facultatifs, un ou plusieurs dérivés d'acide salicylique, un ou plusieurs coupleurs et un ou plusieurs solubilisants.

On connaît, certes, de EP-0 738 510 une émulsion huile-dans-eau à effet anti-âge renfermant un dérivé d'acide salicylique et un éther de myristyle polyoxypropyléné. On connaît par ailleurs de WO 95/03779 des compositions cosmétiques à base d'acide salicylique, qui contiennent des esters d'acides gras éthoxylés. La demande EP-0 765 658 divulgue également des compositions cosmétiques à base de dérivés d'acide salicylique, comprenant de l'octyl dodécanol. De même, la demande EP-0 615 748 décrit des matrices pour la formation de microsphérules destinées à protéger des actifs sensibles à l'oxydation, tels que l'acide acétylsalicylique. Ces microsphérules sont notamment formées à partir d'alcools gras ramifiés. Aucune de ces compositions ne contient toutefois de coupleur au sens de la présente invention.

Par ailleurs, le brevet US-5,382,432 divulgue des compositions à base d'acide salicylique comprenant notamment un coupleur constitué de glycérides capryliqueslcapriques oxyéthylénés. De même, la demande GB-2 283 421 divulgue l'utilisation d'un alcool gras ou d'une huile oxyéthylénés pour solubiliser un actif anti-acné tel que l'acide salicylique, éventuellement en association avec des coupleurs constitués d'esters de glycéryle oxyéthylénés. Toutefois, les compositions divulguées dans ces deux documents ne contiennent pas de solubilisant au sens de la présente invention.

L'invention a donc pour objet une composition comprenant de l'eau, au moins un dérivé d'acide salicylique, au moins un solubilisant et au moins un coupleur,
caractérisée en ce que ledit solubilisant est choisi parmi :
(a) un composé de la formule:

   R(OCH(CH₃)CH₂)ₙOH

   où R est un groupe aliphatique saturé ou un groupe aliphatique non saturé contenant une ou plusieurs doubles liaisons, linéaire, ramifié ou cyclique en Ci-C₂₂, et n varie de 2 à 22, et
(b) un composé de la formule :

   RCH(R')CH₂OH
où R est un groupe aliphatique saturé ou non saturé, linéaire ou ramifié, ayant 1 à 18 atomes de carbone, et R' est un groupe aliphatique saturé ou non saturé, linéaire, ramifié ayant 2 à 12 atomes de carbone,
et en ce que ledit coupleur est choisi parmi :
(a') un dérivé de polyéthylène glycol d'un ou d'un mélange de mono-, di- et triglycérides d'acides en C₄ à C₁₆ avec une moyenne de 2 à 12 moles d'oxyde d'éthylène,
(b') un ester PEG-n-glycéryle de formule :

   RCOOCH₂CH(OH)CH₂(OCH₂CH₂)ₙOH

   où R est un groupe aliphatique saturé ou non saturé linéaire ou ramifié ayant 2 à 24 atomes de carbone et où n est 3 à 40,
(c') un alkylpolyglucoside..

Le dérivé d'acide salicylique de l'invention comprend des composés de la formule I et leurs sels topiquement acceptables : 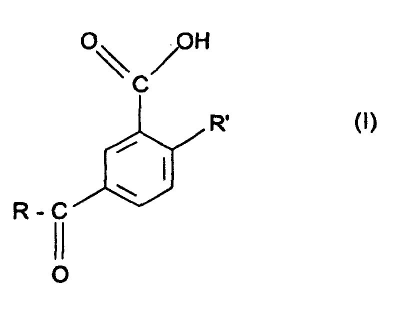 où R est un groupe aliphatique saturé ou un groupe aliphatique non saturé linéaire, ramifié ou cyclique, le groupe non saturé contenant une ou plusieurs doubles liaisons qui peuvent ou non être conjuguées, ces groupes contenant 2 à 22 atomes de carbone et pouvant être remplacés par au moins un substituant choisi parmi (a) les atomes halogène, (b) le groupe trifluorométhyle, (c) les groupes hydroxyle sous forme libre ou estérifiée par un acide ayant 1 à 6 atomes de carbone ou (d) un groupe fonctionnel carboxyle qui est libre ou estérifié par un alcool inférieur ayant 1 à 6 atomes de carbone ;
R' est un groupe hydroxyle ou un groupe fonctionnel d'ester possédant la formule suivante : 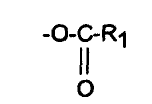 où R₁ est un groupe aliphatique saturé ou non saturé linéaire ou ramifié ayant 1 à 18 atomes de carbone.

En outre, les dérivés d'acide salicylique particulièrement préférés, utiles dans la présente invention, comprennent l'acide n-octanoyl-5 salicylique ou acide capryloyl salicylique, l'acide n-décanoyl-5 salicylique, l'acide n-dodécanoyl-5 salicylique, l'acide n-heptyloxy-5 salicylique et l'acide n-heptyloxy-4 salicylique. Un dérivé d'acide salicylique particulièrement préféré est l'acide capryloyl salicylique (appellation commerciale : Mexoryl SAB), voir page 139 de l'International Cosmetic Ingredient Dictionary, 6e édition, Volume I, publié par la Cosmetic Toiletries and Fragrance Association, 1995.

Dans la formule I ci-dessus, le groupe R contient de 2 à 22 atomes de carbone, comprenant chaque atome de carbone dans cette fourchette, y compris les sous-fourchettes. Les numéros de carbone utiles sont 4, 6, 8, 10, 12, 14, 16 et 18. Pour le groupe R' de la formule I ci-dessus, chaque numéro de carbone entre 1 et 18 est spécifiquement inclus, comme le sont toutes les sous-fourchettes. Les numéros de carbone utiles sont 2, 4, 6, 8, 10, 12, 14 et 16. Tous les numéros de carbone impairs entre les atomes de carbone 2 et 22 pour R, et tous les numéros de carbone impairs entre 1 et 18 pour R' sont aussi spécifiquement inclus.

On peut obtenir des sels utiles du dérivé d'acide salicylique de l'invention par salification à l'aide d'une base. Les bases utiles comprennent les bases inorganiques telles que les alcalis et les hydroxydes de métaux alcalins (hydroxyde de sodium, hydroxyde de potassium, etc.) ou les hydroxydes d'ammonium. On peut également utiliser des bases organiques aux fins de la salification. Les bases amphotères sont aussi utiles. On peut se référer à la demande U.S. No. de série 08/627 965, incorporée ici par référence, pour les dérivés utiles de l'acide salicylique et les sels utiles de ceux-ci. Les sels quaternaires tels que les sels de diméthylhydroxypropylammonium sont aussi particulièrement utiles.

Les solutions, les compositions, les formulations, les mélanges, etc. de la présente invention peuvent comprendre un ou un mélange des dérivés d'acide salicylique décrits ci-dessus. Généralement, il est préférable que les dérivés d'acide salicylique de l'invention soient présents dans les solutions, les mélanges, les formulations, les compositions, les gels, etc. de l'invention dans des quantités allant de 0,1 à 65 % en poids par rapport au poids total de la composition, de préférence de 1 à 30 % en poids, plus préférablement 2 % en poids ou plus comprenant 3, 4, 5, 6, 7, 8, 9, 10, 12, 14, 16, 18, 20, 22, 24, 26, 28 pour cent en poids, etc.

Le solubilisant de la présente invention a une affinité lipophile légèrement plus élevée que les coupleurs de l'invention et sert à solubiliser le dérivé d'acide salicylique de l'invention en une forme liquide et à accroître sa biodisponibilité pour la peau. Bien qu'on puisse utiliser l'octyldodécanol (Eutanol G) et des alcools linéaires et ramifiés C₁₀-C₃₀ similaires, la préférence est donnée aux composés de la formule suivante :

R(OCH(CH₃)CH₂)ₙOH

où R est un groupe aliphatique saturé ou un groupe aliphatique non saturé contenant une ou plusieurs doubles liaisons, linéaire, ramifié ou cyclique en C₁-C₂₂. n varie de 2 à 22. Les solubilisants préférés sont ceux conformes à la formule susvisée où R est un groupe en C₈, C₁₀, C₁₂, C₁₄, C₁₆ ou C₁₈, et n est 3, 4, 5, 6, 7, 8, 9, 10, 11 ou 12. R est de préférence un radical alkyle linéaire ou ramifié, et plus préférablement un radical alkyle linéaire. Ces composés peuvent être généralement décrits comme des éthers d'alkyle de polypropylène glycol (éthers d'alkyle de PPG) et peuvent également être désignés comme éthers PPG-n-C- où les variables n et C correspondent aux variables n et R dans la formule susvisée.

Des exemples préférés spécifiques des solubilisants de l'invention comprennent l'éther PPG-3-myristylique et l'éther PPG-10-cétylique. Se reporter à l'International Cosmetic Ingredient Dictionary, 6e édition, Volume 1, CTFA, 1995, pour de plus amples précisions concernant ces solubilisants et des solubilisants apparentés. Le groupe cétyle correspond à R=C₁₆ dans la formule susvisée, le groupe myristyle correspond à C₁₄.

Dans la présente invention, on peut utiliser un mélange d'un ou de plusieurs solubilisants, et le ou les solubilisants sont typiquement présents dans des quantités allant de 0,1 à 50 % en poids, de préférence de 1 à 20 % en poids, et encore plus préférablement de 2 à 10 % en poids, le tout par rapport au poids total de la composition, y compris 3, 4, 5, 6, 7, 8 et 9 % en poids.

Comme mentionné ci-dessus, les solubilisants tels que l'octyldodécanol (voir pages 637-638 de l'international Cosmetic Ingredient Dictionary, 6e édition, Volume 1, CTFA, 1995, incorporé ici par référence) peuvent être employés bien que les éthers d'alkyle de PPG susmentionnés soient préférés. Les solubilisants utiles comprennent donc les composés de la formule :

RCH(R')CH₂OH

où R est un groupe aliphatique saturé ou non saturé, linéaire ou ramifié, ayant 1 à 18, de préférence 4 à 12, plus préférablement 6 à 10 atomes de carbone, et R' est un groupe aliphatique saturé ou non saturé, linéaire, ramifié, ayant 2 à 12, préférablement 4 à 10, le plus préférablement 6 à 8 atomes de carbone. Ces solubilisants peuvent être utilisés seuls, dans des mélanges de deux ou plus, ou dans des mélanges comprenant des éthers d'alkyle de PPG indiqués ci-dessus. La quantité totale de solubilisant est comme décrit ci-dessus, indépendamment de la présence de solubilisants tels que l'octyldodécanol.

Les coupleurs de l'invention sont généralement des émollients hydrosolubles qui présentent une grande affinité pour l'eau tout en conservant un fort caractère lipophile. Ces caractéristiques permettent le couplage entre l'eau, le solubilisant et le dérivé d'acide salicylique de la présente invention. Les coupleurs utiles dans la présente invention comprennent les dérivés de polyéthylène glycol de mono-, di- et triglycérides d'acides en C₄ à C₁₆ avec une moyenne de 2 à 12 moles d'oxyde d'éthylène, les esters PEG-n-glycéryle qui correspondent à la formule générale :

RCOOCH₂CH(OH)CH₂(OCH₂CH₂)ₙOH

où R est un groupe aliphatique saturé ou non saturé linéaire ou ramifié ayant 2 à 24, préférablement 4 à 20, plus préférablement 6 à 18 atomes de carbone et où n représente la valeur moyenne des unités de polyéthylène glycol et va de 3 à 40, et les alkylpolyglucosides. Ces dernières substances sont préférées car elles offrent d'excellents résultats et elles sont plus douces que les substances éthoxylées de coupleurs.

Les coupleurs utiles dans la présente invention comprennent, particulièrement, les glycérides capryliques/capriques de PEG-8 et les glycérides capryliques/capriques de PEG-6. En outre, d'autres glycérides d'alkyle de PEG peuvent être utilisés, ayant, généralement, de 2 à 12 moles d'oxyde d'éthylène et une chaîne comportant de 4 à 16, préférablement de 6 à 14, plus préférablement 8, 10 ou 12 atomes de carbone. Se reporter aux définitions des glycérides capryliques/capriques de PEG-6 et des glycérides capryliques/capriques de PEG-8 aux pages 682 et 683 de l'International Cosmetic Ingredient Dictionary, 6e édition, Volume 1, 1995, CTFA.

Un autre type de coupleur qui peut être utilisé dans la présente invention est les esters PEG-n-glycéryle qui correspondent à la formule générale :

RCOOCH₂CH(OH)CH₂(OCH₂CH₂)ₙOH

où R est un groupe aliphatique saturé ou non saturé linéaire ou ramifié ayant 2 à 24, préférablement 4 à 20, plus préférablement 6 à 18 atomes de carbone et où n représente la valeur moyenne des unités de polyéthylène glycol et va de 3 à 40. Des exemples précis de ces substances sont le cocoate de glycéryle PEG-7, le cocoate de glycéryl PEG-8, etc. Les valeurs n préférées dans la formule susvisée varient entre 3 et 40, y compris 5, 10, 15, 20, 25 et 30. Se reporter aux pages 696 à 701 de l'international Cosmetic Ingredient Dictionary, 6e édition, Volume 1, CTFA.

Ces esters PEG-n-glycéryle peuvent être utilisés en combinaison avec les acides gras en C₆ à C₂₄ et un éther de polyéthylène glycol de glycérine avec une valeur d'éthoxylation moyenne de 7 à 20. Voir Glycereth-7, -12 et -20 à la page 401 de l'Intemational Cosmetic Ingredient Dictionary sus-identifié.

Les coupleurs particulièrement préférés dans la présente invention sont les alkylpolyglucosides tels que le caprylyl/capryl-glucoside et le décylglucoside. Se reporter aux pages 139 et 286 de l'International Cosmetic Ingredient Dictionary sus-identifié. D'autres exemples des glucosides d'alkyle utiles dans la présente invention sont analogues au décylglucoside et comprennent les produits obtenus par la condensation des alcools en C₅ à C₂₄, avec un polymère de glucose. Les numéros de carbone de l'alcool de 6 et plus sont préférés. De même, des composés similaires au caprylyl/capryl-glucoside ayant la formule : 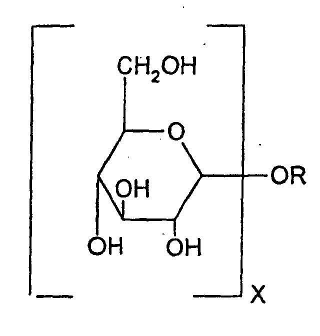 où R représente un groupe aliphatique saturé ou non saturé, linéaire ou ramifié, ayant 4 à 22 atomes de carbone, préférablement 6 à 18 atomes de carbone, y compris 8, 10, 12, 14 et 16 atomes de carbone, peuvent être utilisés. X peut varier et n'est pas limité. Les structures polymères sont préférées.

Le composant du coupleur de l'invention peut comprendre plus d'une catégorie des coupleurs susmentionnés et peut comprendre des mélanges de différents coupleurs du même type.

Préférablement, le coupleur de l'invention est présent en une quantité allant de 5 à 75 % en poids, par rapport au poids total de la composition, préférablement de 15 à 50 % en poids, y compris 20, 25, 30, 35, 40 et 45 % en poids, ainsi que 55, 60, 65 et 70 % en poids, et la quantité de coupleur(s) est de préférence ajustée de manière à fournir un produit transparent et stable.

Les solutions, les mélanges, les formulations, les compositions, les gels, etc. de la présente invention contiennent de l'eau en des quantités allant de préférence de 1 à 94,8 % en poids, mieux 10 à 90 %, comprenant 20, 30, 40, 50, 60, 70, 75, 80 et 85 % en poids par rapport au poids total de la composition. De manière plus préférée, l'eau représente au moins 40 % en poids et plus, le plus de préférence 50 à 90 % en poids par rapport au poids total de la composition. L'eau utilisée est, de préférence, pure ou obtenue à partir de sources, etc. connues dans le monde entier comme ayant la réputation de fournir de la bonne eau. Selon un mode préféré de réalisation, on utilise de l'eau désionisée.

Le pH des solutions, compositions, formulations, mélanges, gels, etc. de la présente invention est, de préférence, ajusté à un niveau final de 0,5 à 11, plus préférablement de 1 à 10, et mieux de 2 à 8, y compris 2,5, 3, 3,5, 4, 4,5, 5, 5,5, 6, 6,5, 7, 7,5 et 8. Toutes les fourchettes et les sous-fourchettes sont comprises. Le pH est acide dans une réalisation très préférée. Les pH variant entre 2 et 3, ou entre 3 et 4, sont les plus préférés. Tout agent conventionnel d'ajustement du pH peut être utilisé pour obtenir le pH final de l'invention, tel que l'acide citrique, l'hydroxyde de potassium, tout tampon connu, etc.

Les compositions de l'invention constituent notamment des compositions topiques, en particulier cosmétiques et/ou dermatologiques. Dans ce cas, les compositions doivent contenir un milieu topiquement acceptable, c'est-à-dire compatible avec la peau, les muqueuses, les ongles, le cuir chevelu et/ou les cheveux.

Suivant la pratique courante des compositions cosmétiques et dermatologiques, d'autres ingrédients peuvent être présents dans n'importe laquelle ou lequel des solutions, formulations, compositions, mélanges, gels, etc., de l'invention. Ces autres composants comprennent des conservateurs, des colorants, des hydratants, des agents actifs tels que médicaments, vitamines, etc., parfum, agents absorbant les UV, agents filtrant les UV, protéines ou hydrolysais de protéine, acides aminés, polyols, urée, alpha-hydroxy-acides, acide salicylique lui-même, sucre, dérivés de sucre, agents gélifiants tels que gomme de xanthane, polymères carboxyvinyliques, dérivés de cellulose, pectines, polyacrylamides, etc. Les agents cosmétiques et pharmacologiques utiles qui peuvent être ajoutés aux solutions, mélanges, compositions, formulations, etc. de l'invention comprennent l'hydrocortisone, l'acide rétinoïque, le rétinol (vitamine A), l'acide ascorbique (vitamine C), la vitamine E et les dérivés de ces vitamines tels que les esters et notamment le palmitate de rétinyle, l'acétate de rétinyle, etc.

Les compositions, les solutions, etc. de l'invention peuvent également contenir des ingrédients qui diminuent les effets irritants des dérivés de l'acide salicylique, tels que les antagonistes de la substance P, les antagonistes de CGRP, etc. Se reporter par exemple aux documents EP-A-680 749, EP-A-716 850, EP-A-723 774, EP-A-737 471 et EP-A-770 392.

Les solutions, les formulations, les mélanges, les compositions, les gels, etc. de l'invention peuvent être réalisés par les personnes de compétence ordinaire dans l'art utilisant des techniques bien connues dans l'art. Une méthode préférée pour préparer les compositions de l'invention consiste à mélanger le solubilisant de l'invention et le dérivé d'acide salicylique, et à ajouter subséquemment ce mélange à une phase aqueuse contenant le coupleur de l'invention. Suivant l'identité du coupleur, sa quantité et la présence éventuelle d'autres ingrédients, les compositions de l'invention peuvent être préparées avec des viscosités similaires à celles de l'eau et plus, y compris des compositions de gel et similaires au gel. Dans les réalisations les plus préférées de la présente invention, les mélanges, les solutions, les formulations, les compositions, les gels, etc. de l'invention sont transparents lors d'une inspection visible et ils sont stables, demeurant de préférence inchangés à température ambiante pendant une période d'au moins 12 semaines sans précipitation, séparation des phases, appréciable, ou perte de clarté. Dans une réalisation hautement préférée, l'invention offre des solutions, formulations, mélanges, compositions, gels, etc. qui subissent peu de changement ou aucun changement lorsque soumis à une température de 45°C pendant douze semaines, à six cycles de gel/dégel ou à des conditions froides telles que 5°C.

Les solutions, les formulations, les mélanges, les compositions, les gels, etc. de la présente invention peuvent également avoir des viscosités inférieures à celles de l'eau, et peuvent prendre la forme de toute formulation cosmétique et dermatologique conventionnelle connue dans l'art, indépendamment de sa viscosité. Ainsi, la présente invention peut prendre la forme d'une émulsion utilisant des émulsifiants et des techniques connus dans l'art pour produire des émulsions. Les émulsions peuvent être des émulsions huile dans eau ou eau dans huile. Les pâtes et les crèmes sont également des formes acceptables pour l'invention. Ces formes et leur production sont bien connues de ceux de compétence ordinaire dans l'art. Voir l'international Cosmetic Ingredient Dictionary, Volumes 1 et 2, 1995, CTFA; pour les émulsifiants, les huiles, les tensioactifs connus, etc.

Les dérivés d'acide salicylique de l'invention sont kératolytiques et ont notamment pour effet de favoriser un renouvellement cellulaire, et ils se sont avérés donner des résultats et des effets qui sont ceux habituellement liés aux alpha-hydroxy-acides. Cependant, on a observé que les dérivés d'acide salicylique de l'invention sont plus actifs et offrent un profil plus efficace que les alpha-hydroxy-acides d'utilisation courante (ainsi, on peut comparer une formulation d'acide capryloyl salicylique à 2 % suivant l'invention à une formulation d'acide lactique à 10 %). Par conséquent, les usages éventuels de la présente invention comprennent tous ceux connus pour les alpha-hydroxy-acides, y compris les usages et les méthodes énumérés dans les brevets US-A-5,547,998, US-A-5,422,370, US-A-5,389,677, US-A-5,385,938, US-A-5,091,171, US-A-5,554,597, US-A-5,554,651, US-A-5,554,652, US-A-5,554,654, US-A-5 556 882, US-A-5 561 153, US-A-5 561 155, US-A-5,561 156, US-A-5 561 157, US-A-5,561,158, US-A-5,561,159, US-A-5,565,487, US-A-5,470,880, US-A-5,643,952, US-A-5,643,953, US-A-5,643,961, US-A-5,643,962, US-A-5,643,963, US-A-5,654,336 et US-A-5,654,340.

Les méthodes d'utilisation des composés de l'invention comprennent donc le traitement et la prévention des signes du vieillissement, le traitement des rides, etc. En outre, l'utilisation pour préparer des compositions dermatologiques destinées à traiter les verrues et l'acné est également incluse. Pour mettre en oeuvre l'invention, la solution, la formulation, le mélange, la composition, le gel, etc. de l'invention sont appliqués à la peau, là où les effets sont désirés. Le traitement peut être une application localisée à un endroit ou une caractéristique particuliers, ou peut être général, y compris l'emplacement de la caractéristique.

Aussi, la présente invention a aussi pour objet un procédé cosmétique de traitement de la peau, consistant à appliquer sur la peau, la composition telle que définie ci-dessus.

La présente invention a encore pour objet l'utilisation de la composition telle que définie ci-dessus pour la préparation d'une composition dermatologique destinée à traiter et/ou à prévenir les verrues et/ou l'acné.

Les exemples suivants sont donnés à titre d'illustration de l'invention.

Les compositions ont été préparées en mélangeant ensemble les ingrédients énumérés. Le solubilisant et l'ingrédient actif ont d'abord été mélangés ensemble, et ce mélange a été ajouté à l'eau en présence d'un coupleur.

**Tableau No. 1**

| Composition | % en poids | % en poids |
|---|---|---|
| **Diluant** | | |
| Eau | 72 | 67 |
| | | |
| **Coupleur** | | |
| L.A.S. | 20 | 25 |
| | | |
| **Solubilisant** | | |
| Promyristyl PM-3® | 5 | |
| Procetyl-10® | | 5 |
| | | |
| **Ingrédient actif** | | |
| Mexoryl SAB® | 2 | 2 |
| | | |
| **Conservateur** | | |
| Germaben II® | 1 | 1 |
| **Total** | 100 | 100 |
| **Aspect** | C | C |
| **pH** | 2,65 | 2,90 |
| L.A.S. : triglycérides capryliques/capriques de PEG-8 (GATREFOSSE) | | |
| Promyristyl PM-3® : éther myristylique de PPG-3 (CRODA) | | |
| Procetyl-10® : éther cétylique de PPG-10 (CRODA) | | |
| Mexoryl SAB® : acide capryloyl salicylique (L'OREAL) | | |
| Germaben II® : mélange de conservateurs (SUTTON) | | |
| Abréviation : C = transparent | | |

**Tableau No. 2**

| Composition | % en poids | % en poids |
|---|---|---|
| **Diluant** | | |
| Eau | 50 | 47 |
| | | |
| **Coupleur** | | |
| Oramix CG-110® (à 60 %) | 42 | |
| Oramix NS-10® (à 55 %) | | 45 |
| | | |
| **Solubilisant** | | |
| Procetyl-10® | 5 | 5 |
| | | |
| **Ingrédient actif** | | |
| Mexoryl SAB® | 2 | 2 |
| | | |
| **Conservateur** | | |
| Germaben II® | 1 | 1 |
| **Total** | 100 | 100 |
| **Aspect** | VCS | VCS |
| **pH** | 3,50 | 3,53 |
| Oramix CG-110® (à 60 %) : caprylyl/capryl glucoside (SEPPIC) | | |
| Oramix NS-10® (à 55 %) : décylglucoside (SEPPIC) | | |
| Procetyl-10® : éther cétylique de PPG-10 (CRODA) | | |
| Mexoryl SAB® : acide capryloyl salicylique (L'OREAL) | | |
| Germaben II® : mélange de conservateurs (SUTTON) | | |
| Abréviation : VCS = très clair et stable | | |

**Tableau No. 3**

| Composition | % en poids | % en poids | % en poids |
|---|---|---|---|
| **Diluant** | | | |
| Eau | 67 | 67 | 67 |
| | | | |
| **Coupleur** | | | |
| Cetiol HE® | 25 | | |
| Glycerox 767® | | 25 | |
| Glycerox HE® | | | 25 |
| | | | |
| **Solubilisant** | | | |
| Procetyl-10® | 5 | 5 | 5 |
| | | | |
| **Ingrédient actif** | | | |
| Mexoryl SAB® | 2 | 2 | 2 |
| | | | |
| **Conservateur** | | | |
| Germaben II® | 1 | 1 | 1 |
| **Total** | 100 | 100 | 100 |
| **Aspect** | TG | C | CLG |
| **pH** | 2,78 | 2,97 | 2,97 |
| Cetiol HE® : cocoate de glycéryle PEG-7/acide gras de coco/Glycereth-7 (HENKEL) | | | |
| Glycerox 767® : triglycérides capriques/capryliques de PEG-6 (CRODA) | | | |
| Glycerox HE® : cocoate de glycéryle PEG-7 (CRODA) | | | |
| Procetyl-10® : éther cétylique de PPG-10 (CRODA) | | | |
| Mexoryl SAB® : acide capryloyl salicylique (L'OREAL) | | | |
| Germaben II® : mélange de conservateurs (SUTTON) | | | |
| Abréviations : TG = gel translucide à basse viscosité | | | |
| C = transparent | | | |
| CLG = gel clair à basse viscosité | | | |

**Tableau No. 4**

| Composition | % en poids | % en poids |
|---|---|---|
| **Diluant** | | |
| Eau | 74,5 | 86,75 |
| | | |
| **Coupleur** | | |
| Oramix CG-110® (à 60 %) | 21 | 10,5 |
| | | |
| **Solubilisant** | | |
| Procetyl-10® | 2,5 | 1,25 |
| | | |
| **Ingrédient actif** | | |
| Mexoryl SAB® | 1 | 0,5 |
| | | |
| **Conservateur** | | |
| Germaben II® | 1 | 1 |
| **Total** | 100 | 100 |
| **Aspect** | Transparent | Transparent |
| **pH** | 3,51 | 3,51 |
| Oramix CG- 1 10® (à 60 %): caprylyl/capryl glucoside (SEPPIC) | | |
| Procetyl-10® : éther cétylique de PPG-10 (CRODA) | | |
| Mexoryl SAB® : acide capryloyl salicylique (L'OREAL) | | |
| Germaben II® : mélange de conservateurs (SUTTON) | | |

**Tableau No. 5**

| Composition | % en poids | % en poids | % en poids | % en poids | % en poids |
|---|---|---|---|---|---|
| **Diluant** | | | | | |
| Eau | 71,17 | 67 | 60,75 | 69,27 | 57,9 |
| | | | | | |
| **Coupleur** | | | | | |
| Oramix CG-110® (à 60%) | 20,83 | 25 | 31,25 | | |
| Oramix NS-10® (à 55%) | | | | 22,73 | 34,1 |
| | | | | | |
| **Solubilisant** | | | | | |
| Procetyl-10® | 5 | 5 | 5 | 5 | 5 |
| | | | | | |
| **Ingrédient actif** | | | | | |
| Mexoryl SAB® | 2 | 2 | 2 | 2 | 2 |
| | | | | | |
| **Conservateur** | | | | | |
| Germaben II® | 1 | 1 | 1 | 1 | 1 |
| **Total** | 100 | 100 | 100 | 100 | 100 |
| **Aspect** | PS | O | C | O | C |
| **pH** | - | 3,46 | 3,71 | 3,48 | 3,29 |
| Oramix CG-110® (à 60 %) : caprylyl/capryl glucoside (SEPPIC) | | | | | |
| Oramix NS-10® (à 55 %) : décylglucoside (SEPPIC) | | | | | |
| Procetyl-10® : éther cétylique de PPG-10 (CRODA) | | | | | |
| Mexoryl SAB® : acide capryloyl salicylique (L'OREAL) | | | | | |
| Germaben II® : mélange de conservateurs (SUTTON) | | | | | |
| Abréviations : | | | | | |
| PS = séparation de phase | | | | | |
| 0 = opaque/translucide | | | | | |
| C = transparent | | | | | |

II va sans dire que l'invention telle que décrite se prête à de nombreuses modifications.
L'invention n'est pas limitée aux réalisations spécifiques décrites ci-dessus.

## Revendications

1. Composition comprenant de l'eau, au moins un dérivé d'acide salicylique qui est un composé de la formule I ou ses sels topiquement acceptables : 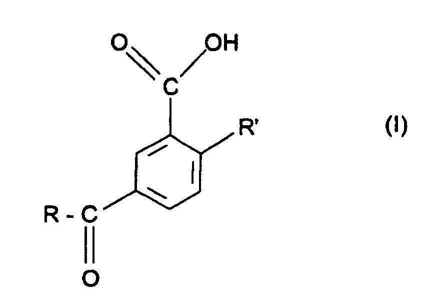 dans laquelle
R est un groupe aliphatique saturé ou un groupe aliphatique non saturé, linéaire, ramifié ou cyclique, le groupe non saturé contenant une ou plusieurs doubles liaisons qui peuvent ou non être conjuguées, ces groupes contenant 2 à 22 atomes de carbone et pouvant être remplacés par au moins un substituant choisi parmi (a) les atomes halogène, (b) le groupe trifluorométhyle, (c) les groupes hydroxyle sous forme libre ou estérifiée par un acide ayant 1 à 6 atomes de carbone ou (d) un groupe fonctionnel carboxyle qui est libre ou estérifié par un alcool inférieur ayant 1 à 6 atomes de carbone ;
R' est un groupe hydroxyle ou un groupe fonctionnel d'ester possédant la formule suivante : 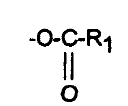 où R₁ est un groupe aliphatique saturé ou non saturé linéaire ou ramifié ayant 1 à 18 atomes de carbone, au moins un solubilisant et au moins un coupleur,
**caractérisée en ce que** ledit solubilisant est choisi parmi :
(a) un composé de la formule :
R(OCH(CH₃)CH₂)ₙOH
où R est un groupe aliphatique saturé ou un groupe aliphatique non saturé contenant une ou plusieurs doubles liaisons, linéaire, ramifié ou cyclique en C₁-C₂₂, et n varie de 2 à 22, et
(b) un composé de la formule:
RCH(R')CH₂OH
où R est un groupe aliphatique saturé ou non saturé, linéaire ou ramifié, ayant 1 à 18 atomes de carbone, et R' est un groupe aliphatique saturé ou non saturé, linéaire, ramifié ayant 2 à 12 atomes de carbone,
et **en ce que** ledit coupleur est choisi parmi :
(a') un dérivé de polyéthylène glycol d'un ou d'un mélange de mono-, di- et triglycérides d'acides en C₄ à C₁₆ avec une moyenne de 2 à 12 moles d'oxyde d'éthylène,
(b') un ester PEG-n-glycéryle de formule :
RCOOCH₂CH(OH)CH₂(OCH₂CH₂)ₙOH
où R est un groupe aliphatique saturé ou non saturé linéaire ou ramifié ayant 2 à 24 atomes de carbone et où n est 3 à 40,
(c') un alkylpolyglucoside.

2. Composition selon la revendication 1, **caractérisée en ce que** ledit dérivé d'acide salicylique est l'acide capryloyl salicylique.

3. Composition selon l'une quelconque des revendications précédentes, **caractérisée en ce que** ledit coupleur est un alkylpolyglucoside obtenu par la condensation d'un alcool en C₅ à C₂₄ avec un polymère de glucose.

4. Composition selon l'une quelconque des revendications précédentes, **caractérisée en ce que** ladite composition comprend de 0,1 à 65 % en poids de dérivé d'acide salicylique, de 0,1 à 50 % en poids de solubilisant, de 5 à 75 % en poids de coupleur, et de 1 à 94,8 % en poids d'eau, le tout par rapport au poids total de la composition.

5. Composition selon l'une quelconque des revendications précédentes, **caractérisée en ce que** ladite composition est sous forme de solution ou de gel.

6. Composition selon l'une quelconque des revendications précédentes, **caractérisée en ce que** ladite composition est transparente.

7. Composition selon l'une quelconque des revendications précédentes, **caractérisée en ce qu'**elle constitue une composition cosmétique et/ou dermatologique.

8. Procédé cosmétique de traitement de la peau, consistant à appliquer sur la peau, la composition selon l'une quelconque des revendications précédentes.

9. Procédé cosmétique de prévention ou de traitement des signes du vieillissement, consistant à appliquer sur la peau la composition selon l'une quelconque des revendications 1 à 7.

10. Utilisation de la composition selon l'une quelconque des revendications 1 à 7, pour la préparation d'une composition dermatologique destinée à traiter et/ou à prévenir les verrues et/ou l'acné.

## Patentansprüche

1. Zusammensetzung, die Wasser, mindestens ein Salicylsäurederivat, bei dem es sich um eine Verbindung der Formel I oder ein topisch akzeptables Salz dieser Verbindungen handelt: 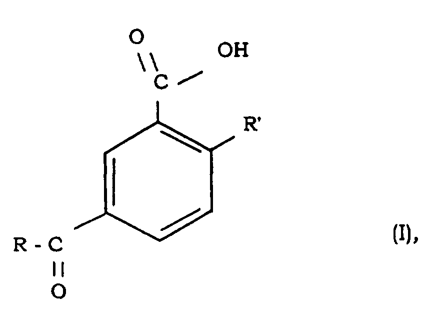 worin bedeuten:
R eine geradkettige, verzweigte oder cyclische, gesättigte aliphatische Gruppe oder eine geradkettige, verzweigte oder cyclische ungesättigte aliphatische Gruppe, wobei die ungesättigte Gruppe eine oder mehrere Doppelbindungen enthält, die konjugiert oder nicht konjugiert sein können, wobei die Gruppen 2 bis 22 Kohlenstoffatome besitzen und wobei bei den Gruppen der folgende Ersatz vorliegen kann: mindestens ein Substituent, der (a) unter den Halogenatomen, (b) der Trifluormethylgruppe, (c) den Hydroxygruppen in freier Form oder verestert mit einer Säure mit 1 bis 6 Kohlenstoffatomen oder (d) einer funktionellen Carboxygruppe, die frei oder verestert mit einem niederen Alkohol mit 1 bis 6 Kohlenstoffatomen vorliegt, ausgewählt ist; und
und dadurch, dass der Kuppler unter den folgenden Verbindungen ausgewählt ist:
(a') einem Polyethylenglykolderivat eines oder eines Gemisches von Mono-, Di- und Triglyceriden von C₄₋₁₆-Säuren mit einem Mittelwert von 2 bis 12 Mol Ethylenoxid,
(b') einem PEG-n-glycerylester der Formel:
RCOOCH₂CH(OH)CH₂(OCH₂CH₂)ₙOH,
worin R eine gesättigte oder ungesättigte, geradkettige oder verzweigte aliphatische Gruppe mit 2 bis 24 Kohlenstoffatomen ist und n im Bereich von 3 bis 40 liegt, und
(c') einem Alkylpolyglucosid.

2. Zusammensetzung nach Anspruch 1, **dadurch gekennzeichnet, dass** das Salicylsäurederivat die Capryloyl-salicylsäure ist.

3. Zusammensetzung nach einem der vorhergehenden Ansprüche, **dadurch gekennzeichnet, dass** der Kuppler ein Alkylpolyglucosid ist, das durch Kondensation eines C₅₋₂₄-Alkohols mit einem Glucosepolymer hergestellt ist.

4. Zusammensetzung nach einem der vorhergehenden Ansprüche, **dadurch gekennzeichnet, dass** die Zusammensetzung 0,1 bis 65 Gew.-% Salicylsäurederivat, 0,1 bis 50 Gew.-% Lösungsvermittler, 5 bis 75 Gew.-% Kuppler und 1 bis 94,8 Gew.-% Wasser enthält, bezogen auf das Gesamtgewicht der Zusammensetzung.

5. Zusammensetzung nach einem der vorhergehenden Ansprüche, **dadurch gekennzeichnet, dass** die Zusammensetzung als Lösung oder Gel vorliegt.

6. Zusammensetzung nach einem der vorhergehenden Ansprüche, **dadurch gekennzeichnet, dass** die Zusammensetzung transparent ist.

7. Zusammensetzung nach einem der vorhergehenden Ansprüche, **dadurch gekennzeichnet, dass** es sich um eine kosmetische und/oder dermatologische Zusammensetzung handelt.

8. Kosmetisches Verfahren zur Behandlung der Haut, das darin besteht, auf die Haut die Zusammensetzung nach einem der vorhergehenden Ansprüche aufzutragen.

9. Kosmetisches Verfahren zur Vorbeugung oder zur Behandlung von Alterszeichen, das darin besteht, auf die Haut die Zusammensetzung nach einem der Ansprüche 1 bis 7 aufzutragen.

10. Verwendung der Zusammensetzung nach einem der Ansprüche 1 bis 7 zur Herstellung einer dermatologischen Zusammensetzung, die dazu vorgesehen ist, Warzen und/oder Akne zu behandeln und/ oder Warzen und/oder Akne vorzubeugen.

## Claims

1. Composition comprising water, at least one salicylic acid derivative which is a compound of the formula (I) or its topically acceptable salts: 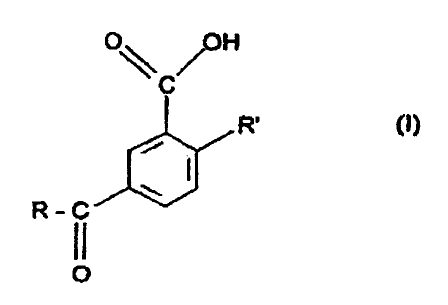 in which
R is a saturated, linear, branched or cyclic, aliphatic group or an unsaturated, linear, branched or cyclic, aliphatic group, the unsaturated group comprising one or more double bonds which may or may not be conjugated, these groups comprising 2 to 22 carbon atoms and being able to be replaced by at least one substituent chosen from (a) halogen atoms, (b) the trifluoromethyl group, (c) hydroxyl groups in the free form or in the form esterified by an acid having 1 to 6 carbon atoms, or (d) a carboxyl functional group which is free or esterified by a lower alcohol having 1 to 6 carbon atoms;
R' is a hydroxyl group or an ester functional group having the following formula:

2. Composition according to Claim 1, **characterized in that** the said salicylic acid derivative is capryloylsalicylic acid.

3. Composition according to either one of the preceding claims, **characterized in that** the said coupling agent is an alkylpolyglucoside obtained by the condensation of a C₅ to C₂₄ alcohol with a glucose polymer.

4. Composition according to any one of the preceding claims, **characterized in that** the said composition comprises from 0.1 to 65% by weight of salicylic acid derivative, from 0.1 to 50% by weight of solubilizer, from 5 to 75% by weight of coupling agent and from 1 to 94.8% by weight of water, all with respect to the total weight of the composition.

5. Composition according to any one of the preceding claims, **characterized in that** the said composition is in the form of a solution or of a gel.

6. Composition according to any one of the preceding claims, **characterized in that** the said composition is transparent.

7. Composition according to any one of the preceding claims, **characterized in that** it constitutes a cosmetic and/or dermatological composition.

8. Cosmetic process for the treatment of the skin, comprising the application to the skin of the composition according to any one of the preceding claims.

9. Cosmetic process for preventing or treating signs of ageing, comprising the application to the skin of the composition according to any one of Claims 1 to 7.

10. Use of the composition according to any one of Claims 1 to 7 in the preparation of a dermatological composition intended to treat and/or to prevent warts and/or acne.
